(19) Europäisches Patentamt

European Patent Office

Office européen des brevets

(11) **EP 0 969 284 A1**

(12) **EUROPÄISCHE PATENTANMELDUNG**

(43) Veröffentlichungstag:
05.01.2000 Patentblatt 2000/01

(51) Int. Cl.$^7$: **G01N 33/542**, G01N 33/50,
C12N 15/85, C12Q 1/68

(21) Anmeldenummer: 99112544.4

(22) Anmeldetag: 01.07.1999

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **02.07.1998 DE 19829495**

(71) Anmelder:
• **Paysan, Jacques**
 **70599 Stuttgart (DE)**
• **Antz, Christof**
 **72070 Tübingen (DE)**

(72) Erfinder:
• **Paysan, Jacques**
 **70599 Stuttgart (DE)**
• **Antz, Christof**
 **72070 Tübingen (DE)**

(74) Vertreter:
 **Fuhlendorf, Jörn, Dipl.-Ing.**
 **Patentanwälte**
 **Dreiss, Fuhlendorf, Steimle & Becker,**
 **Postfach 10 37 62**
 **70032 Stuttgart (DE)**

(54) **Untersuchung von Wechselwirkungen zwischen zellulären Molekülen und deren Lokalisation in Zellen**

(57) Ein fluorogener Vektor zur Fluoreszenzmarkierung spezifischer Ziele in lebenden Zellen besteht aus einem Membrantranslokationspeptid, einer Spezifizierungskomponente (Peptid oder Antikörper) und einer Fluorophore. Anwendungen sind Fluoreszenzmikroskopie, Studium von intrazellulären Wechselwirkungen durch FRET-Messung und Screeningsverfahren.

EP 0 969 284 A1

Printed by Xerox (UK) Business Services
2.16.7/3.6

**Beschreibung**

Die vorliegende Erfindung betrifft einen fluorogenen Vektor und einen Reagentiensatz zur Fluoreszenzmarkierung spezifischer Ziele in Zellen.

Die vorliegende Erfindung betrifft ebenfalls deren Anwendung zur Lokalisierung von Molekülen und zur Identifizierung und Analyse von Wechselwirkungen zwischen solchen Molekülen.

Die Visualisierung von zellulären Molekülen (z. B. Proteinen, Ionen, Lipiden etc.) durch Fluoreszenzmikroskopie ist eine wichtige Methode der zellbiologischen Forschung. Die Entwicklung neuartiger Fluoreszenzfarbstoffe, die als Sensoren für zelluläre Vorgänge wie Ionenbewegungen oder molekularen Wechselwirkungen dienen können, hat in den letzten Jahren zur Weiterentwicklung dieser Technologien beigetragen.

Die Absorptionsspektren und Fluoreszenzpektren sind charakteristisch für die Fluorophorenmoleküle und durch deren molekulare Architektur bestimmt. In der Fluoreszenzmikroskopie nutzt man die Stokesche Verschiebung, um fluoreszierende Moleküle sichtbar zu machen. Dazu beleuchtet man das zu untersuchende Objekt z. B. mittels eines Lasers oder einer Quecksilberhochdrucklampe durch einen Anregungsfilter, der Licht nur bis zu einer möglichst genau definierten Wellenlänge passieren läßt. Gleichzeitig betrachtet man die entstehende Fluoreszenzstrahlung über einen dichromatischen Spiegel und einen Emissionsfilter, sodass reflektiertes oder gestreutes Anregungslicht abgeblockt und nur das Fluoreszenzlicht sichtbar wird. Zur Messung können außer optischen Mikroskopen auch Photomultiplier oder gekühlte CCD-Kameras verwendet werden.

Unter "Quenching" versteht man den Effekt, bei dem die von der Fluorophore emittierte Energie nicht als Photon in Erscheinung tritt, sondern von einem benachbarten Molekül, dem "Quencher", absorbiert wird. Dazu ist es notwendig, dass die Absorptionsbanden des Quenchers signifikant mit den Emissionsbanden der Fluorophore überlappen. Eine besondere Form des Quenching tritt dann auf, wenn der Quencher selbst ein fluoreszierendes Molekül ist. In diesem Fall spricht man von Fluoreszenz-Resonanz-Energie-Transfer (FRET), wobei die erste Fluorophore als FRET-Donor (D) und der Quencher als FRET-Akzeptor (A) bezeichnet werden.

Wenn beispielsweise der Donor ein Absorptionsmaximum im ultravioletten Bereich des Spektrums aufweist und blaues Fluoreszenzlicht emittiert, dann hat ein dazu passender FRET-Akzeptor ein Absorptionsmaximum im blauen Bereich des Spektrums, während er z. B. grüne oder gelbe Fluoreszenz abstrahlt. Tritt FRET zwischen den beiden Komponenten auf, dann absorbiert das Donor-Akzeptor-Paar im ultravioletten Bereich und emittiert grüne bzw. gelbe Fluoreszenz. Durch eine geeignete Auswahl von dichromatischen Spiegeln und Filtern kann so FRET unabhängig von der Fluoreszenz der einzelnen Komponenten betrachtet werden.

Die Wahrscheinlichkeit, dass zwischen einem geeigneten Fluorophorenpaar D und A FRET auftritt, nimmt stark mit dem räumlichen Abstand zwischen den beiden Fluorophoren ab. Man bezeichnet den Abstand, bei dem 50% der angeregten Donormoleküle über FRET "gelöscht" werden als Försterradius.

Für die Fluorophoren Fluorescein und Tetramethylrhodamin beträgt der Försterradius ungefähr 55Å. Durch die starke Abhängigkeit der FRET-Wahrscheinlichkeit vom Abstand zwischen den zwei beteiligten Fluorophoren kann FRET zur Messung molekularer Abstände verwendet werden. Nach Clegg (Clegg et al., Fluorescence Resonance Energy Transfer. In Fluorescence Imaging Spectroscopy and Microscopy. Wiley, New York 1996, S. 179-252) wird der Abstand zwischen zwei Fluorophoren nach der Beziehung $1 - F_{DA}/F_D = 1/(1+(r/r_0))^6$ berechnet. Dabei sind $F_{DA}$ und $F_D$ die Fluoreszenz des Donors in An- und Abwesenheit des Akzeptors, $r_0$ der Försterradius und r die zu bestimmende Distanz.

Die Technik der subzellulären Lokalisierung von Epitopen durch fluoreszenzmarkierte Antikörper ist in der Immunfluoreszenzmikroskopie bekannt. Meistens wird dabei ein unmarkierter Primärantikörper an ein zelluläres Epitop gebunden und dann durch eine fluoreszenzmarkierten Sekundärantikörper detektiert. Viele Firmen bieten kommerziell ein breites Angebot solcher Sekundärantikörper an, die mit einer Vielzahl ebenfalls kommerziell erhältlicher Fluoreszenzfarbstoffe gekoppelt sein können.

Die Anwendung der Immunfluoreszenz zur Analyse der dynamischen Lokalisation von Molekülen in lebenden Zellen und zur Analyse der intrazellulären Wechselwirkungen dieser Moleküle durch FRET ist aber im wesentlichen auf extrazelluläre Epitope beschränkt. Der Grund hierfür ist, dass fluoreszenzmarkierte Antikörper hydrophile Moleküle sind, die die Zellmembran lebender Zellen nicht durchqueren können. Immunfluoreszenz an intrazellulären Epitopen wird deshalb an fixierten Zellen durchgeführt, deren Zellmembran durch Detergentien permeabilisiert wurde. Es ist deshalb von größtem Interesse für die zellbiologische Forschung, fluoreszierende Substanzen zu entwickeln, die spezifisch an intrazelluläre Moleküle binden und die in lebende Zellen eingeschleußt werden können.

Die Klonierung des "Green-Fluorescent-Protein" (GFP) aus der pazifischen Qualle *Aequoria victoria* (Prasher et al., Gene 111(2):229-233, 1992) hat der Lokalisierung von Proteinen (Marshall et al., Neuron 14(2):211-215, 1995) und Organellen (Rizzuto et al., Curr Biol 5(6):635-642, 1995; De Giorgi et al., Gene 173: 113-117, 1996) in lebenden Zellen durch Fluoreszenzmikroskopie neue Perspektiven eröffnet. GFP ist ein 238 Aminosäuren langes Protein mit einer äußerst stabilen, faßförmigen Struktur (Ormö et al., Science 273: 1392-1395, 1996). GFP fluoresziert ohne zusätzliche Cofaktoren und zwar auch dann, wenn es in heterologen Zellen (z. B. Säugerzellen, Insektenzellen, Bakterien, Hefe etc.) exprimiert wird oder völlig aus seinem zellulären Umfeld extrahiert wird. Dadurch, dass GFP ein genetisch exprimierbares und modifizierbares Molekül darstellt kann es als fluoreszierendes Modul in rekombinante Proteinchimären eingebaut werden. Es sind bereits Beispiele bekannt, bei denen GFP in Zielproteine eingebaut wurde, um deren subzelluläre Verteilung und Dynamik in lebenden Zellen studieren zu können. So wurde z. B. die Verteilung von L-Typ und Non-L-Typ $Ca^{2+}$-Kanälen in Muskelzellen analysiert (Grabner et al., PNAS 95: 1903-1908, 1998), die subzelluläre Verteilung von Hydroxymethylglutaryl-CoA Reduktase in Hefezellen (Hampton et al., PNAS 93: 828-833, 1996) und die dynamische Verteilung des Glukocortikoidrezeptors in Mäusezellen (Htun et al., PNAS 93: 4845-4850, 1996). Eines der eindrucksvollsten Beispiele ist die Erzeugung einer transgenen Maus, in der Actin mit GFP fusioniert vorliegt (Andrew Matus, Biozentrum in Basel). In diesem Tier kann die Dynamik des Actin-Cytoskeletts in lebendem, intaktem Gewebe fluoreszenzmikroskopisch beobachtet werden.

Durch Mutationen im Bereich der Fluorophore von GFP kann eine Veränderung der Spektraleigenschaften des Proteins erreicht werden (Heim und Tsien, Current Biology 6: 178-182, 1996; US Patent # 5,625,048)). Dabei sind besonders Mutationen von Interesse, die zu den Spektralvarianten BFP (Blue-Fluorescent-Protein), CFP (Cyan-Fluorescent-Protein) und YFP (Yellow-Fluorescent-Protein) geführt haben, sowie die Mutation S65T, die einen singulären Absorptionspeak für GFP bei 489nm hervorbringt. Damit stehen zwei Paare von GFP-Varianten (BFP/GFP und CFP/YFP) zur Verfügung, die als Donor/Akzeptor-Paare für FRET geeignet sind und somit auch zur Analyse von Proteinwechselwirkungen verwendet werden können.

Eine Anwendung dieser Paare wurde in der WO97/28261 vorgeschlagen. Sie beschreibt ein Tandemprotein, in dem BFP und GFP (Chamäleon-1), bzw. CFP und YFP (Chamäleon-2) durch eine Peptidkette verbunden sind, die aus Calmodulin und dem Calmodulin-bindenden Peptid M13 besteht (Miyawaki et al, Nature 388(6645):882-887, 1997). In Anwesenheit von $Ca^{2+}$-Ionen bindet Calmodulin an M13 und induziert dadurch eine intramolekulare Konformationsänderung der gesamten Proteinchimäre. Donor (BFP oder CFP) und Akzeptor (GFP oder YFP) ändern infolge dieser Umlagerung ihren Abstand zueinander und es kommt zu FRET, dessen Quantifizierung zur Messung der intrazellulären $Ca^{2+}$-Konzentration verwendet werden kann. Durch geeignete Signalsequenzen, die den Chamäleon-Chimären angeheftet werden, ist es möglich diese $Ca^{2+}$-Sensoren gezielt in Zellorganellen zu dirigieren und so Calciummessungen in definierten subzellulären Kompartimenten vorzunehmen. In ähnlicher Weise konnte die selbe Arbeitsgruppe pH-Sensoren entwickeln, mit denen der intrazelluläre pH-Wert im Cytoplasma, den Mitochondrien und im Golgiapparat getrennt gemessen werden kann (Llopis et al., PNAS 95: 6803-6808, 1998).

Herman und Mitarbeiter haben gezeigt (Mahajan et al., Nature Biotechnology 16 (6): 547-552, 1998), dass die physiologische Wechselwirkung der Proteine Bcl-2 und Bax dargestellt werden kann, indem man die Proteine als Fusionsproteine mit GFP und BFP in Säugerzellen exprimiert und FRET zwischen den BFP- und GFP-Anteilen der Chimären misst.

Trotz dieser Erfolge stößt die Verwendung von GFP und dessen Spektralvarianten für die subzelluläre Lokalisierung von Proteinen und besonders für die Analyse von Proteinwechselwirkungen durch FRET aber rasch auf Grenzen. Ein Nachteil bei der Verwendung von GFP und dessen Spektralvarianten für die Fluoreszenzmikroskopie und für FRET-Messungen ist, dass diese Verfahren auf exprimierbare Molekülklassen beschränkt sind. Ein weiterer Nachteil beruht auf der Struktur und Größe des GFP. GFP ist ein Protein mit einer stabilen, faßförmigen Struktur, die als β-barrel bezeichnet wird (Phillips, Current Opinion in Structural Biology 7: 821-827, 1997). In Fusionchimären mit anderen Proteinen kann sich dieses "Faß" durch eine massive Störung der nativen Proteinstruktur bemerkbar machen und stellt eine erhebliche Beeinträchtigung vieler Protein/Protein-Wechselwirkungen dar.

Die Erfinder der vorliegenden Anmeldung haben z. B. erkannt, dass eine Fusion von GFP mit dem C-Terminus von $P2X_2$-Rezeptoren zu einer membranständigen Fusionschimäre führt, wenn dieses Konstrukt in NG-108 Zellen exprimiert wird. Andererseits verliert der $P2X_2$-Rezeptor seine Membranlokalisierung in den selben Zellen, wenn das GFP mit dem N-Terminus des Proteins verknüpft wird. Die N-terminale Chimäre bleibt im Syntheseapparat der Zellen stecken (eigene, unveröffentlichte Ergebnisse). Die Gründe hierfür sind unbekannt.

Darüber hinaus stellen die Spektren der verschiedenen GFP-Mutationen brauchbare, bisher aber keine idealen Donor/Akzeptor-Paare zur Verfügung. Die Emissionsspektren des Donors fallen in der Regel flach ab und der dadurch bedingte "tail" des Donoremissionsspektrums reicht bis weit in das Emissionsspektrum des Akzeptors hinein. Dadurch kommt es zu einem Durchstrahlen der Donorfluoreszenz in den Spektralbereich, in dem FRET beobachtet und gemessen wird. Dadurch sind aufwendige Kontrollen und eine sorgfältige Auswertung zur quantitativen Analyse von FRET erforderlich.

Es ist das Ziel der vorliegenden Erfindung, fluorogene Vektoren der eingangs genannten Art anzubieten, die in lebenden, funktionsfähigen Zellen verwendet werden können, an möglichst vielartige intrazelluläre Ziele binden können, ohne diese selbst störend zu beeinträchtigen und bestmögliche spektrale Eigenschaften aufweisen. Diese Aufgabe löst ein Vektor gemäß Patentanspruch 1, der aus wenigstens einer Membrantranslokationskomponente, einer Spezifizierungskomponente und einer fluorophoren Komponente besteht.

Die drei Komponenten des erfindungsgemäßen Vektors und deren Anwendungen werden nun näher erläutert. Bevorzugte Ausführungsformen des Vektors und Anwendung ergeben sich aus den Unteransprüchen und Anwendungsansprüchen.

1.) Die Fluoreszenz-Komponente. Diese Komponente ist bevorzugt eine Fluorophore (z. B. FITC, Rhodamin usw.), die kovalent oder nichtkovalent an die zweite Komponente gekoppelt ist. Statt einem Fluoreszenzfarbstoff ist im Spezialfall auch eine nichtfluoreszierende Chromophore denkbar, die lichtmikroskopisch visualisiert werden kann.

Die Verwendung von fluoreszenzmarkierten Antikörpern als FRET-Donoren oder -Akzeptoren bringt gegenüber GFP/BFP- oder CFP/YFP-Paare einen erheblichen Vorteil. Die Fluorophoren werden über den Antikörper indirekt an das Zielmolekül angeheftet. Das Zielmolekül selber bleibt also unverändert. Der Antikörper kann dabei (in gewissen Grenzen) durch die Wahl des Epitops auf verschiedene Stellen des Zielmoleküls dirigiert werden, ohne dessen Struktur a priori zu stören. Die Antikörper können ex vivo mit beliebigen Fluoreszenzfarbstoffen markiert werden, wozu eine breite Palette von Fluorophoren zur Verfügung steht, deren Spektren z. B. schärfere Maxima und eine günstigere Stokesche Verschiebung aufweisen als GFP.

Mit den momentan kommerziell erhältlichen Fluorophoren kann praktisch das gesamte sichtbare Spektrum lückenlos abgedeckt werden (Siehe z. B. Handbook of Fluorescent Probes and Research Chemicals, 6th Ed., Molecular Probes, 1996) und die so zur Verfügung stehenden Fluorophoren haben meist eine weit höhere Quantenausbeute als GFP. So kann eine ideale Donor/Akzeptor-Kombination ausgewählt und verwendet werden. Außerdem können Antikörper auch gegen nicht exprimierbare Molekülklassen gerichtet werden (z. B. Kohlenhydrate, Lipide etc.), die als Chimären mit GFP kaum denkbar sind.

2.) Die Spezifizierungs-Komponente. Darunter ist eine molekulare Komponente zu verstehen, die die fluorogenen Vektoren spezifisch an ein subzelluläres Ziel dirigiert. Unter einem subzellulären Ziel ist bevorzugt ein definiertes Molekül, bzw. eine Molekülklasse zu verstehen, aber auch subzelluläre Kompartimente und Organellen, wie Endosomen, Lysosomen, endoplasmatisches Retikulum, Golgi-Apparat, Synapsen etc.

Die Spezifizierungskomponente kann erfindungsgemäß ein Peptid sein, das spezifisch an ein Zielmolekül, z. B. ein Protein bindet. Hierbei sind auch sogenannte Signalpeptide gemeint, die zu einer selektiven Lokalisierung in definierte subzelluläre Kompartimente führen, z. B. in den Golgiapparat (Siehe z. B. Roth und Berger, Journal of Cell Biology 93: 223-229, 1982) oder die Mitochondrien (Siehe z. B. Hurt et al., EMBO Journal 4: 2061-2068, 1985). Besonders ist an Peptide gedacht, die durch geeignete Screeningverfahren und Optimierungsstrategien identifiziert wurden. Unter geeigneten Screeningverfahren sind Verfahren zu verstehen, wie das Screening von "phage-display" und "yeast two-hybrid" Bibiotheken, deren Gebrauch dem Fachmann geläufig ist und die kommerziell erhältlich sind. Mit diesen Methoden können kurze, kombinatorische Peptide identifiziert werden, die spezifisch an zelluläre Proteine binden. Die Bindungsaffinität und -spezifität solcher Peptide kann optimiert werden, z. B. in einem rekursiven Verfahren, das Strukturanalyse durch NMR-Spektroskopie, wiederholte gezielte Mutagenese und Affinitäts- und Spezifitätsanalysen umfasst. Statt der Peptide ist auch an Antikörper gedacht, bzw. Antikörperfragmente und rekombinante Einzelstrangantikörper, wie sie wiederum durch "phage-display" Klonierung isoliert werden können (Winter et al., Annual Reviews in Immunology 12: 433, 1994). Die Spezifizierungskomponente kann auch ein Protein sein, ein Proteinfragment, ein synthetisches Peptid oder ein Peptoid, sowie jeder andere, an die Markierungskomponente koppelbare Molekülanteil, der zu einer spezifischen subzellulären Lokalisierung des Partikels führt.

3.) Die Membrantranslokations-Komponente. Hierunter ist eine Molekülkomponente zu verstehen, die zur Einschleusung der fluorogenen Vektoren durch die Zellmembran in lebende Zellen führt. Bestimmte Proteinabschnitte aus Homeodomänenproteinen weisen hierfür geeignete Eigenschaften auf. Die Helix 3 aus Antennapedia zum Beispiel (einem Transkriptionsfaktor aus Drosophila melanogaster) durchquert die Membranen von lebenden Zellen und zwar ohne dafür Stoffwechselenergie zu verbrauchen und ohne, dass hierfür ein spezifischer Rezeptor auf der Zelloberfläche notwendig ist (Derossi et al., Trends in Cell Biology 8: 84-87, 1998; Prochiantz, Current Opinion in Neurobiology 6: 629-634, 1996).

Besonders geeignet erscheinen die Variationen der 16 Aminosäure-Reste umfassenden Sequenz 43-58 des Homeodomänen-Proteins Antennapedia, wie sie in einer Veröffentlichung von Alain Prochiantz beschrieben sind (Current Opinion in Neurobiology 6: 629-634 (1996)), z.B. RQIKIWFQNRRMKWKK (43->58) und RQIKIWFPNRRMKWKK (Pro50). Ebenfalls geeignet sind von dieser Sequenz abgeleitete Peptide, wie sie in der WO 97/12912 beschrieben sind, bzw.

andere Peptide mit ähnlichen Eigenschaften, wie sie z. B. von Rojas beschrieben wurden (Rojas et al., Nature Biotechnology 16: 370-375 (1998)).

Man vermutet, dass die Peptide mit geladenen Phospholipiden auf der Membranaußenseite interagieren, dadurch den Membranbilayer lokal destabilisieren und so invertierte Micellen bilden (Derossi et al., Journal of Biological Chemistry 271: 18188-18193 (1996)). Diese Micellen durchqueren dann die Membran, öffnen sich auf der cytosolischen Seite und entlassen ihren Inhalt ins Zellinnere. Eine Variante der Helix 3 wird als Penetratin durch Appligene Oncor (Illkirch, Frankreich) verkauft, in einer aktivierten Form, die eine chemische Kopplung an Zielpeptide ermöglicht.

Es gibt noch weitere Versuche, synthetische Peptide mit Translokationseigenschaften zu konzipieren (Journal of Peptide Research 51: 235-243, 1998).

Unter fluorogenen Vektoren sind molekulare Anordnungen zu verstehen, in denen Fluoreszenz-Komponente, Spezifizierungs-Komponente und Membrantranslokations-Komponente kovalent oder nicht-kovalent verbunden werden.

Aufgabe der fluorogenen Vektoren ist es, fluoreszierende Markierungen (im Sonderfall auch nichtfluoreszierende, aber durch Lichtmikroskopie visualisierbare Chromophoren) an spezifische Ziele in lebenden Zellen anzuheften. Dabei ist es erfindungsgemäß unerheblich, ob nach dem Eindringen in die Zelle die Translokationskomponente intrazellulär abgespalten wird oder nicht. Maßgeblich ist nur, dass in der lebenden Zelle die Spezifizierungskomponente und die Fluoreszenzkomponente räumlich assoziiert bleiben, um so eine fluoreszenzmikroskopisch messbare Markierung an einem spezifischen zellulären Ziel anzubringen. Diese Fluoreszenzmarkierung kann dann für die Analyse der dynamischen subzellulären Verteilung des Zielmoleküls in lebenden Zellen benutzt werden, oder durch FRET zwischen zwei, eventuell mehreren unterschiedlichen und gemeinsam in die Zellen eingebrachten fluorogenen Vektoren eines Reagentiensatzes, zur Analyse von Wechselwirkungen zellulärer Moleküle. Dabei kann im Spezialfall der FRET Donor oder der Akzeptor auch eine Fusionschimäre mit Varianten des GFP darstellen, die seperat in den Zellen exprimiert wird.

Letzteres kann zum Beispiel in Screeningverfahren Anwendung finden. Dazu kann zum Beispiel ein hefeoptimiertes GFP-Gen (Cormack et al., Microbiology 143: 303-311, 1997) in einem induzierbaren Expressionsvektor (z. B. pYES2, Invitrogen) mit einer Vielzahl von DNA-Sequenzen verbunden werden, die verschiedene natürliche oder kombinatorische Proteinanteile kodieren. Die so entstandene Bibliothek von Genen, die GFP/Protein bzw. GFP/Peptid-Chimären kodieren werden dann in Hefezellen transformiert und die Expression der Hybridproteine induziert. Dann werden die Zellen in einer Lösung von Rhodamin- oder AMCA-markierten fluorogenen Vektoren inkubiert, die als Spezifizierungskomponente ein Zielmolekül, z. B. ein Protein, Proteinfragment, Peptid, Peptoid etc. enthalten. Die Wechselwirkung des Zielmoleküls mit den GFP-Chimären wird durch Messung von FRET bestimmt, wobei GFP als Donor und Rhodamin als Akzeptor, bzw. AMCA als Donor und GFP als Akzeptor fungiert. Erfindungsgemäß verwendbare Farbstoffe sind z. B. folgende Substanzen, die unter den Handelsbezeichnungen BODIPY-Farbstoff, Fluorescein, Oregon Green, Rhodol Green, Rhodamin, Texas Red, Cy2, Cy3, Cy5, Alexa, Marina Blue, Pacific Blue, AMCA etc. dem Fachmann bekannt sind. Die Analyse von FRET findet z. B. durch einen fluoreszenzaktivierten Zellsorter (FACS) oder durch FRET-Mikroskopie statt.

Dieses Screeningsverfahren kann ebenfalls für Bakterienzellen, sowie Eukaryontenzellen, wie Insektenzellen, Amphibienzellen und Säugerzellen angewandt werden.

## Patentansprüche

1. Fluorogener Vektor zur Fluoreszenzmarkierung spezifischer Ziele in Zellen, der aus wenigstens einer Membrantranslokationskomponente, einer Spezifizierungskomponente und einer fluorophoren Komponente besteht.

2. Vektor nach Anspruch 1, dadurch gekennzeichnet, dass die Membrantranslokationskomponente ein Peptid mit Membrantranslokationseigenschaften ist.

3. Vektor nach Anspruch 2, dadurch gekennzeichnet, dass das genannte Peptid ein Abschnitt eines homeodomänen Proteins ist.

4. Vektor nach Anspruch 3, dadurch gekennzeichnet, dass das genannte Peptid eine Variation der 16 Aminosäure-Reste umfassenden Sequenz 43-58 des Homeodomäns Antennapedia (Antp) ist.

5. Vektor nach Anspruch 4, dadurch gekennzeichnet, dass das Peptid RQIKIWFQNRRMKWKK oder RQIKIWFPNRRMKWKK (Pro50) ist.

6. Vektor nach einem der vorhergehenden Ansprüche, dadurch gekennzeichnet, dass die Spezifizierungskomponente ein Peptid, ein Proteinfragment oder ein Protein ist.

7. Vektor nach einem der Ansprüche 1 bis 5, dadurch gekennzeichnet, dass die Spezifizierungskomponente zwischen Antikörper, rekombinanten Einzelstrangantikörpern und Antikörperfragmenten ausgewählt wird.

8. Vektor nach Anspruch 6 oder 7, dadurch gekennzeichnet, dass die Spezifizierungskomponente mittels eines "Phage Display"-Verfahren ausgewählt wird.

9. Vektor nach Anspruch 6 oder 7, dadurch gekennzeichnet, dass die genannte Spezifizierungskomponente durch ein "Yeast-2-hybrid"-Verfahren ausgewählt wird.

10. Vektor nach Anspruch 8 oder 9, dadurch gekennzeichnet, dass die Spezifizität und Bindungsaffinität der genannten Spezifizierungskomponente durch ein rekursives Verfahren optimiert wird, welches wiederholte gezielte Mutagenese umfasst.

11. Vektor nach einem der vorhergehenden Ansprüche, dadurch gekennzeichnet, dass die fluorophore Komponente fähig ist, Donor oder Akzeptor für Fluoreszenz-Resonanz-Energie-Transfer (FRET) gegenüber einem fluoreszierenden Protein zu sein.

12. Vektor nach Anspruch 11, dadurch gekennzeichnet, dass das fluoreszierende Protein ein rekombinantes Fusionsprotein mit GFP oder eine seiner Spektralvarianten ist.

13. Reagentiensatz für FRET-Messungen, dadurch gekennzeichnet, dass er wenigstens zwei fluorogene Vektoren nach einem der Ansprüche 1 bis 12 enthält, wobei die fluorophoren Komponenten der Vektoren ein FRET-Donor/Akzeptor-Paar bilden.

14. Reagentiensatz nach Anspruch 13 zur Messung der Wechselwirkungen zwischen zwei Substanzen, dadurch gekennzeichnet, dass die Spezifizierungskomponente des ersten Vektors diesen ersten Vektor auf die erste Substanz dirigiert und die Spezifizierungskomponente des zweiten Vektors diesen zweiten Vektor auf die zweite Substanz dirigiert.

15. Anwendung eines Vektors bzw. Reagentiensatzes nach einem der Ansprüche 1 bis 14 zur Detektion eines Zieles in einer biologisch funktionsfähigen Zelle, wobei die Spezifizierungskomponente des Vektors auf das genannte Ziel dirigiert ist.

16. Anwendung eines Vektors nach einem der Ansprüche 11 oder 12 zur FRET-Messung in biologisch funktionsfähigen Zellen, von rekombinanten Fusionsproteinen mit dem genannten fluoreszierenden Protein, wobei die Spezifizierungskomponente auf eine Sequenz des rekombinanten Fusionsproteins dirigiert ist.

17. Anwendung nach Anspruch 16 für ein Screening-Verfahren, wobei das Gen des genannten fluoreszierenden Proteins in einem Expressionsvektor mit anderen codierenden Sequenzen verbunden wird, in einer Zellkultur einem Transformations- und Expressionsverfahren unterworfen wird, die Zellkultur mit dem fluorogenen Vektor inkubiert wird und anschließend einer Trennung unterworfen wird.

18. Anwendung nach Anspruch 17, dadurch gekennzeichnet, dass die Trennung mittels eines Zellsorters oder durch FRET-Mikroskopie ausgeführt wird.

19. Anwendung nach Anspruch 17, dadurch gekennzeichnet, dass das fluoreszierende Protein GFP ist und die fluoreszierende Komponente des fluorogenen Vektors zwischen BODIPY-Farbstoff, Fluorescein, Oregon Green, Rhodol Green, Rhodamin, Texas Red, Cy2, Cy3, Cy5, Alexa, Marina Blue, Pacific Blue, AMCA ausgewählt wird.

20. Anwendung nach einem der Ansprüche 16 bis 19, dadurch gekennzeichnet, dass die lebenden Zellen zwischen Bakterienzellen und Eukaryontenzellen, insbesondere Hefezellen, Insektenzellen, Amphibienzellen und Säugerzellen ausgewählt werden.

21. Anwendung eines Reagentiensatzes nach Anspruch 13 oder 14, zur Messung der Wechselwirkung zwischen zwei, eventuell mehreren Substanzen in lebenden Zellen.

Europäisches
Patentamt

**EUROPÄISCHER RECHERCHENBERICHT**

Nummer der Anmeldung

EP 99 11 2544

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | WO 97 27212 A (RIGEL PHARMACEUTICALS INC) 31. Juli 1997 (1997-07-31) * Seite 50, Zeile 5 - Zeile 30 * --- | 1 | G01N33/542 G01N33/50 C12N15/85 C12Q1/68 |
| D,A | WO 97 12912 A (CENTRE NAT RECH SCIENT ;CHASSAING GERARD (FR); PROCHIANTZ ALAIN (F) 10. April 1997 (1997-04-10) * das ganze Dokument * --- | 1-6 | |
| P,A | WO 99 12033 A (RUPPERSBERG PETER ;ANTZ CHRISTOF (DE); HERLITZE STEFAN (DE); PAYSA) 11. März 1999 (1999-03-11) * das ganze Dokument * ----- | 1-21 | |

| RECHERCHIERTE SACHGEBIETE (Int.Cl.7) |
|---|
| G01N C12Q |

Der vorliegende Recherchenbericht wurde für alle Patentansprüche erstellt

| Recherchenort | Abschlußdatum der Recherche | Prüfer |
|---|---|---|
| DEN HAAG | 24. September 1999 | Gundlach, B |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 99 11 2544

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-09-1999

| Im Recherchenbericht angeführtes Patentdokument | Datum der Veröffentlichung | Mitglied(er) der Patentfamilie | Datum der Veröffentlichung |
|---|---|---|---|
| WO 9727212 A | 31-07-1997 | AU 1707497 A<br>AU 1707897 A<br>CA 2244222 A<br>EP 0877752 A<br>WO 9727213 A | 20-08-1997<br>20-08-1997<br>31-07-1997<br>18-11-1998<br>31-07-1997 |
| WO 9712912 A | 10-04-1997 | FR 2739621 A<br>EP 0797589 A<br>JP 10510557 T | 11-04-1997<br>01-10-1997<br>13-10-1998 |
| WO 9912033 A | 11-03-1999 | DE 19737562 A<br>AU 9437598 A | 06-05-1999<br>22-03-1999 |

EPO FORM P0461

Für nähere Einzelheiten zu diesem Anhang : siehe Amtsblatt des Europäischen Patentamts, Nr.12/82